Europäisches Patentamt

European Patent Office

Office européen des brevets

(11) Publication number: **0 479 619 A1**

## (12) EUROPEAN PATENT APPLICATION

(21) Application number: **91309144.3**

(22) Date of filing: **04.10.91**

(51) Int. Cl.⁵: **A61B 10/00,** C12M 1/30, A61M 31/00, A61F 13/38

(30) Priority: **05.10.90 GB 9021647**

(43) Date of publication of application:
**08.04.92 Bulletin 92/15**

(84) Designated Contracting States:
**BE CH DE ES FR IT LI NL**

(71) Applicant: **Houang, Elizabeth Tingsu**
**22 Horbury Crescent**
**London, W11 3NF (GB)**

(72) Inventor: **Houang, Elizabeth Tingsu**
**22 Horbury Crescent**
**London, W11 3NF (GB)**

(74) Representative: **Waldren, Robin Michael**
**Marks & Clerk, 57-60 Lincoln's Inn Fields**
**London WC2A 3LS (GB)**

(54) Swab.

(57) A swab (1), e.g. for intra-vaginal use, comprises a probe (2) having a contact part (11) for contacting a chosen region of internal body tissue and a sheath (5) for preventing the contact part (11) from touching tissue whilst the swab is being inserted into and withdrawn from the body, the probe (2) and sheath (5) being configured to allow the contact part (11) to be brought into contact with said chosen region when the probe is inserted to a desired extent. The probe comprises at least two telescoped segments (3, 7). At least two of the probe and the telescoped segments, as appropriate, are held in fixed relation to each other by a respective frangible member (15/19).

FIG 1

This invention relates to a swab for insertion into a body orifice when it is important for that part of the swab which is intended to contact deep tissue not to come into contact with tissue nearer the body exterior. In the context of the present invention, the term 'swab' includes both a device for taking a sample and a device intended as an applicator for applying a substance such as a medicament.

The swab of the present invention is particularly suited as a speculum-free high vaginal swab. High vaginal swabs are frequently used in gynaecological examination. The conventional technique of obtaining a specimen is to insert a vaginal speculum and to pass a swab under vision into the upper part of the vagina and withdraw it without touching the lower parts. This is necessary because results obtained from the upper and lower vagina are often different from each other.

The conventional technique described above is time-consuming and subjects the patient to physical discomfort and causes apprehension, particularly if she is pregnant. The doctor or nurse also needs training to pass the vaginal speculum. The result is that there is a tendency to avoid use of a high vaginal swab, even if it would aid confirmation of a clinical diagnosis.

It is known to provide a swab or sampling device on the end of a probe, slidable within a tubular sheath, such as described in the following patent documents: GB 1 394 925; WO 87/02877; US 3 592 186; and US 4 628 941.

It is also known to make such tubular sheaths in the form of two telescoped portions, as described in GB 1 604 477, US 4 235 244 and US 4 023 559. However, with the devices according to the latter disclosures, in each case the means for preventing the swab contact part from contamination before use is complicated and difficult to manufacture.

I have now devised a swab which obviates the use of a speculum when used in intra-vaginal applications and which overcomes the problem of known tubular devices, the swab comprising a probe having a contact part for contacting a chosen region of internal body tissue and a sheath for preventing the contact part from contacting tissue whilst the swab is being inserted into and withdrawn from the body, the sheath comprising at least two telescoped segments and the probe and sheath being configured to allow the contact part to be brought into contact with said chosen region when the probe is inserted to a desired extent and wherein the at least two telescoped segments and/or the probe and the sheath, as appropriate, are held in fixed relation to each other by a respective frangible member.

Thus, (i) the at least two telescoped segments may be held in fixed relation by a frangible member, or (ii) the probe and sheath may be held in fixed relation by a frangible member, or both (i) and (ii) may apply. In case (ii) the at least two telescoped segments may be held together by friction (ie. interference) fit. In case (i), when there are more than two telescoped segments, just two, or more than two of them may be held together by the frangible member.

In most preferred embodiments, the swab is manufactured so as to be disposable, ie. by virtue of being made from low-cost material. The swab is also preferably made of biodegradable material such as cardboard or biodegradable plastics.

It is also preferred that the contact part should comprise absorbent means. For example, it may be an absorbent member such as a cotton bud or a region of the probe integral with the remainder may be fabricated so as to have an absorbent surface.

In the intra-vaginal application, the swab of the present invention can be used to swab the upper parts of the vagina without contamination by the lower parts. Avoidance of such contamination is further avoided because the sheath comprises at least two telescoped segments. Most preferably, these segments comprise a first outer segment for insertion into the vagina and a second inner segment slidably inserted inside the outer segment and housing the contact part before use. If the inner segment is longer than the outer segment, then the contact part can be prevented in use from coming into contact with the contaminated tip of the outer segment.

The swab of the present invention can also be used by the patient herself and to this end it is desirable that the swab be provided with a stop to prevent insertion beyond the desired extent, preferably, this stop is integral with the probe. Prior to use, integrity of the swab is maintained if the probe is attached to the sheath by a further frangible member.

The present invention will now be described by way of a preferred embodiment, with reference to the accompanying drawings in which:-

Figure 1 shows a cross section view of the swab according to the present invention, prior to use;

Figure 2 shows a cross section view of the swab shown in Figure 1 after breaking off a frangible member; and

Figure 3 shows a cross section view of the swab shown in Figures 1 and 2 after breaking a second frangible member.

The speculum free high vaginal swab 1 shown in the figures comprises a probe 2 and a sheath 5. The sheath comprises a first outer waxed cardboard tube 3 open at both ends and a second inner waxed cardboard tube 7 of a smaller diameter, also open at both ends. Instead of cardboard, the cardboard tubes may be made from plastics material, for example a biodegradable plastics material. The probe comprises a stick 9 having a cotton bud 11 attached to one end. At the other end of the stick is attached a stopper 13. The second tube 7 is a tight slidable fit inside the first tube 3.

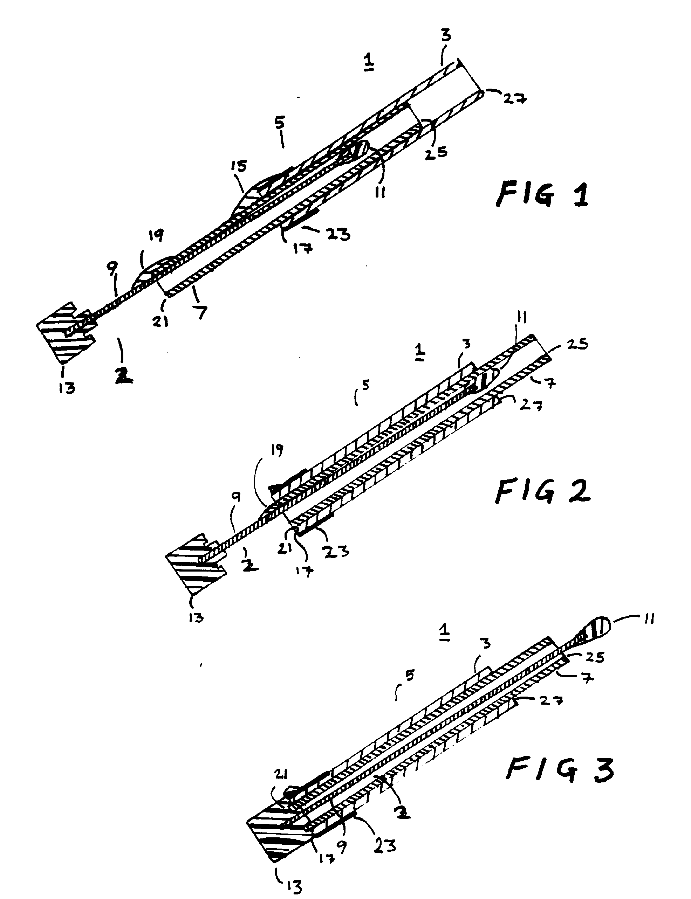

Prior to use, as shown in Figure 1, the first and second tubes are maintained in fixed relation by a frangible seal 15 which can be wax, adhesive, adhesive tape or any other suitable medium joining the outer surface of second tube 7 to one end 17 of the first tube 3. Where the frangible seal is a tape or band, it may for example be frangible by virtue of its thinness or be provided with suitable scoring or perforation. Yet again, it may be the means by which it is bonded to the tubes which is frangible.

The cotton bud is kept inside the tubes, protected from contamination, by virtue of another frangible seal 19 attaching the stick of the probe to one end 21 of the second tube 7. The seal 19 may be composed of any of the materials and in any of the forms mentioned above as suitable for forming the seal 15.

The whole swab should be sterilised by a suitable method and conveyed to the user in appropriate aseptic packaging. In use, the user would remove the swab from the packaging and use it in the following manner.

The first outer tube 3 is held at its lower region 23 which is preferably marked to indicate where it is to be held. Additionally or alternatively, the lower region 23 could be provided with means to assist gripping. The outer tube is inserted into the vagina and relative rotational (twisting) movement between the inner and outer tubes is effected to break the seal 15. The inner tube is then pushed so that end 21 remote from end 25 is level with end 17 of the outer tube, remote from end 27, as shown in Figure 2. This may at least partly fracture the other seal 19.

If the seal 19 is not already completely broken, twisting movement between stopper 13 and the inner tube 7 is then used to detach the probe and the stopper is pushed until it touches end 21 of the inner tube as shown in Figure 3. At this point, the cotton bud contacts the upper vaginal wall so that the required sample is deposited thereon. Up to this point the tubes have protected the cotton bud from contamination from the lower vaginal wall. Also, since the end 25 of the inner tube is clear of the end 27 of the outer tube, by virtue of the inner tube being the longer of the two, the cotton bud cannot be contaminated by contact with end 27 of the outer tube which has touched the lower vaginal wall during insertion.

The probe is then withdrawn and placed in a container containing transport medium so that the cotton bud is kept moist and the sample so preserved. This container is preferably also provided inside the asceptic pack.

When the probe is withdrawn, because the tubes are still in place in the vagina, the bud cannot be contaminated by touching the lower vaginal wall. The two tubes are subsequently withdrawn together and discarded.

The precise dimensions of the parts of the swab are not critical but suitable sizes can easily be determined and in any event may depend on the particular application and the age group of the patient. Typically, the inner tube will be 5-20mm longer than the outer tube and the probe will be 5-20mm longer than the inner tube. In one example, the outer tube is 10mm in outside diameter and 80mm in length, the inner tube is 8mm in outside diameter and 100mm in length, and the probe including the stick and cotton bud but excluding the stopper is 130mm in length. Other variations and embodiments within the scope of the appended claims will now readily be apparent to those skilled in the art.

## Claims

1. A swab (1) comprising a probe (2) having a contact part (11) for contacting a chosen region of internal body tissue and a sheath (5) for preventing the contact part from touching tissue whilst the swab is being inserted into and withdrawn from the body, the sheath comprising at least two telescoped segments (3, 7) and the probe and sheath being configured to allow the contact part (11) to be brought into contact with said chosen region when the probe is inserted to a desired extent, characterised in that the at least two telescoped segments (3, 7) and/or the probe (2) and the sheath (5), as appropriate, are held in fixed relation to each other by a respective frangible member (15/19).

2. A swab according to claim 1, wherein the at least two telescoped segments are held in fixed relation to each other by a frangible member (15).

3. A swab according to either preceding claim, wherein the probe is attached to the sheath by a frangible member (19).

4. A swab according to any preceding claim, wherein the or at least one of the frangible members comprises adhesive.

5. A swab according to any preceding claim, wherein the material from which the sheath is fabricated comprises cardboard.

FIG 1

FIG 2

FIG 3

European Patent
Office

**EUROPEAN SEARCH REPORT**

Application Number

EP    91 30 9144

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 586 604 (ALTER) <br> * column 3, line 29 - column 4, line 32; figures 2-5 * <br> --- | 1,2 | A61B10/00 <br> C12M1/30 <br> A61M31/00 <br> A61F13/38 |
| Y | GB-A-2 028 868 (P.D.C.) <br> * page 3, line 42 - line 52; figures 4-6 * <br> --- | 1,2 | |
| A | US-A-4 175 008 (WHITE) <br> * column 6, line 42 - line 44; figures 1-3 * <br> --- | 4 | |
| A | US-A-2 847 000 (NIEBURGS) <br> * column 2, line 9 - line 10; figures 1,2 * <br> --- | 5 | |
| A | US-A-3 513 830 (KALAYJIAN) <br> --- | | |
| A | US-A-3 800 781 (ZALUCKI) <br> --- | | |
| A,D | US-A-4 235 244 (ABELE) <br><br> ----- | | |

TECHNICAL FIELDS
SEARCHED (Int. Cl.5)

A61B
A61F

The present search report has been drawn up for all claims

| Place of search | Date of completion of the search | Examiner |
|---|---|---|
| THE HAGUE | 02 DECEMBER 1991 | KLEIN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)